Europäisches Patentamt

European Patent Office

Office européen des brevets

(19)

(11) Veröffentlichungsnummer : **0 159 453**
**B1**

(12) **EUROPÄISCHE PATENTSCHRIFT**

(45) Veröffentlichungstag der Patentschrift :
07.06.89

(51) Int. Cl.⁴ : **A 61 B 17/30, B 25 G   1/10**

(21) Anmeldenummer : 84810189.5

(22) Anmeldetag : 16.04.84

(54) Chirurgisches Instrument mit Handgriff.

(43) Veröffentlichungstag der Anmeldung :
30.10.85 Patentblatt 85/44

(45) Bekanntmachung des Hinweises auf die Patenterteilung : 07.06.89 Patentblatt 89/23

(84) Benannte Vertragsstaaten :
CH DE FR GB IT LI SE

(56) Entgegenhaltungen :
FR--A-- 2 451 803
GB--A-- 2 035 187
US--A-- 2 488 484
US--A-- 4 147 443
MARTIN: "Microsurgery-set developed by Vickers-
Owen", 1981, DE;

(73) Patentinhaber : OSCOBAL AG
Bohnackerweg 1
CH-2545 Selzach (CH)

(72) Erfinder : Foucher, Guy, Dr.
4, Bld du Pdt Edwards
F-67000 Strasbourg (FR)
Erfinder : Merle, Michel, Prof.
21, Rue Viriot
F-54410 La Neuveville Devant Nancy (FR)
· Erfinder : Sutter, Hermann
Steinmatten 28
D-7803 Gundelfingen (DE)
Erfinder : Leu, Beat
Kirschbaumallee 11
CH-2563 Ipsach (CH)

(74) Vertreter : Seehof, Michel et al
c/o AMMANN PATENTANWAELTE AG BERN
Schwarztorstrasse 31
CH-3001 Bern (CH)

**Beschreibung**

Die Erfindung betrifft ein zweischenkliges chirurgisches Instrument mit beweglichem Arbeitsende gemäss Oberbegriff von Anspruch 1.

Vor allem bei Instrumenten für die Mikrochirurgie ist die Gefahr eines Abrutschens der haltenden Hand während der sehr diffizilen Arbeit, die unter Umständen sogar unter Vergrösserungs-Optiken stattfinden muss, äusserst nachteilig und muss möglichst ausgeschaltet werden. Somit verlangen bisherige Handgriffe bei der Handhabung der Instrumente eine hohe Konzentration, was vor allem bei länger dauernden Operationen gelegentlich nicht durchzuhalten ist oder eine unzumutbare Anstrengung für den Operateur bedeutet. Eine Verbesserung der Instrumentenhandhabung könnte somit auch die Sicherheit und Qualität der Operation insbesondere bei der Mikrochirurgie verbessern.

Aus der DE-GM 80 25 521 ist ein chirurgisches Instrument mit beweglichen Arbeitsenden, d. h. eine Pinzette gemäss dem Oberbegriff von Anspruch 1 bekannt, die an ihren beiden Pinzettenschenkeln jeweils diese aussen umfassende, die Innenseite freilassende Kunststoffschalen hat, die am Ende der Pinzette miteinander verbunden und im übrigen mit den Pinzettenschenkeln verpresst sind, wodurch sich ein erheblicher Montageaufwand ergibt. Darüber hinaus sind solche Schalengriffe teuer, da sie sehr genau gefertigt sein müssen, um dann mit den eigentlichen Pinzettenschenkeln in einem bestimmten Verhältnis zum Zusammenpressen zusammenzupassen. Dennoch ist eine Trennung der Pinzettenschenkel aus diesen Kunststoffschalen heraus nicht auszuschliessen, wenn beispielsweise die Pinzettenschenkel ausserhalb der Kunststoffschalen erfasst und zusammengedrückt werden und die Kunststoffschalen dann der Bewegung der Pinzettenschenkel nicht folgen. Diese Gefahr ist zumindest im Laufe der Zeit nach mehreren Sterilisierungsvorgängen zu befürchten, wenn die Pressverbindung zwischen den Kunststoffschalen und den Pinzettenschenkeln gelockert ist, weil Kunststoff und Metall unterschiedliche Wärmedehnungsverhalten haben.

Aus der US-A-4 147 443 ist ein chirurgisches Instrument mit einem Handgriff aus weichem Kunststoff bekannt, wobei es sich jedoch um ein Instrument mit einem unbeweglichen Arbeitsende handelt. Der Handgriff wird jeweils um den Metallteil gegossen und die dort offenbarte Aufgabe besteht hauptsächlich darin, eine möglichst gute Verbindung zwischen dem Handgriff und dem Metallteil herzustellen. Ein solcher Handgriff ist jedoch für eine Pinzette nicht verwendbar, da dann deren Funktionsfähigkeit, insbesondere deren Zusammendrückbarkeit, nicht mehr gewährleistet wäre.

Es ist demgegenüber Aufgabe der vorliegenden Erfindung, ein zweischenkliges chirurgisches Instrument zu schaffen, dessen Handgriff, unter Beibehaltung der vollen Funktionsfähigkeit, preiswert herstellbar ist, gut und fest mit dem Instrument verbunden sein oder werden kann und vor allem eine gute Griffigkeit auch bei Verwendung feuchter Gummihandschuhe behält, so dass beliebige, unter Umständen auch grosse Kräfte übertragen werden können und eine geringe Ermüdung eintritt.

Die Lösung dieser Aufgabe ist in den Patentansprüchen gegeben.

Nachstehend ist die Erfindung anhand der Zeichnung von Ausführungsbeispielen näher beschrieben. Es zeigt:

Figur 1 eine Draufsicht eines chirurgischen Instrumentes mit weichem Handgriff, wobei die eine Handgriffhälfte fehlt,

Figur 2 eine Seitenansicht des chirurgischen Instrumentes, teilweise im Schnitt,

Figur 3 einen Längsschnitt durch einen Handgriff und dessen Längsöffnung zum Aufstecken auf einen Pinzettenschenkel,

Figur 4 einen Querschnitt durch eine Handgriffhälfte gemäss der Linie IV-IV in Figur 1.

Beim dargestellten Ausführungsbeispiel eines chirurgischen Instrumentes, nämlich eine Pinzette 1, ist ein Handgriff 3 aus weichem, sterilisierbarem Werkstoff vorgesehen und am Griffbereich 4 des jeweiligen Instrumentes 1 befestigt. Bei der Pinzette 1 besteht der Handgriff 3 aus zwei Griffhälften 3a und 3b. Dabei wäre es möglich, dass der weiche Handgriff 3 am Griffbereich 4 angegossen oder anvulkanisiert ist, wobei der Griffbereich 4 wie im gezeichneten Beispiel einen gleichbleibenden Querschnitt aufweisen oder sich verjüngen kann. Im Ausführungsbeispiel ist der Handgriff 3 jedoch vorgefertigt und auf das jeweilige Instrument 1 bzw. dessen Griffbereich 4 aufsteckbar und lösbar damit verbunden. Das eigentliche, in der Regel aus Metall bestehende Instrument und der Handgriff 3 aus weichem Werkstoff können also getrennt voneinander hergestellt und dennoch sehr einfach verbunden werden.

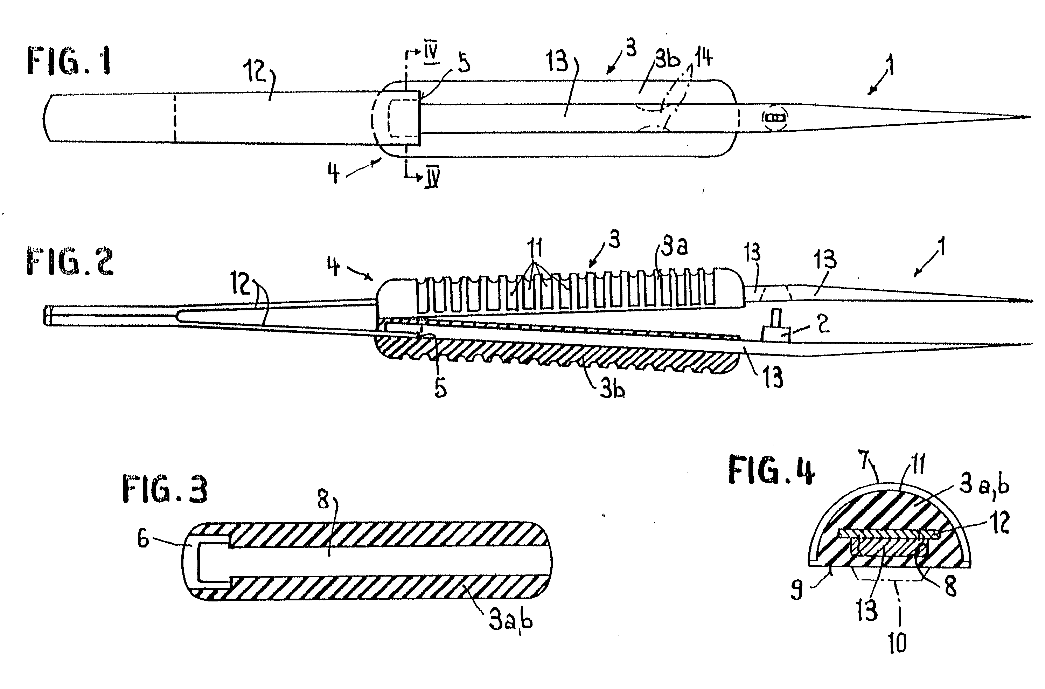

In Figur 1 ist angedeutet, dass der Pinzettenschenkel seine Breite und/oder Dicke ändert, beispielsweise durch Aneinandersetzen zweier unterschiedlicher Flachstücke. Dabei kann ein rückwärtiger Federteil 12 und ein vorderer Arbeitsteil 13 an die jeweiligen Zwecke angepasst und im Bereich des Anschlages 5 verbunden sein. Die Innenöffnung 8 der zugehörigen Griffhälfte 3a, 3b, hat nun eine entsprechende Form, was vor allem in den Figuren 2 bis 4 deutlich wird. Man erkennt, wie die Einstecköffnung 8 an einem Ende eine Ausnehmung 6 hat, an der aber die Höhe dieser Oeffnung 8 geringer wird. Dies kommt der Form der beiden Teile 13 und 12 des Pinzettenschenkels entgegen. Gleichzeitig ergibt sich beim Aufstecken in diesem Bereich ein Anschlag und eine Verrastung des Handgriffes 3 an der Pinzette. Die gute Anpassbarkeit der verschiedenen Bereiche dieses Pinzetteninstrumentes an ihre jeweiligen Zwecke erfüllt in diesem Falle also eine Doppelfunktion, indem sie auch zur Befestigung des Handgriffes 3 beiträgt.

Bei dem in Figur 1 dargestellten zweischenkligen Instrument, der Pinzette 1, ist die jeweilige Handgriffhälfte 3a, 3b für jeden Pinzettenschenkel gemäss Figur 4 im Querschnitt etwa halbkreisförmig. Er könnte aber auch oval oder an seiner Aussenseite 7 vieleckig oder sternförmig sein. Die Durchstecköffnung 8 für den Pinzettenschenkel und den Griffbereich 4 des Instrumentes 1 verläuft dabei nahe der innenseitigen Oberfläche 9. Dadurch können die Schenkel der Pinzette 1 trotz der sie vollständig umhüllenden Griffhälften 3a, 3b eng zusammengedrückt werden. In der Figur 4 ist dabei noch angedeutet, dass an den einander zugewandten Innenflächen 9 der weichen Handgriffe 3 für eine Pinzette 1 Vorsprünge oder Noppen 10 als Abstandhalter vorgesehen sein können, wobei es genügen kann, wenn nur an einem der beiden Griffhälften 3a, 3b einer Pinzette solche Vorsprünge 10 angebracht sind. Diese Vorsprünge 10 verhindern, dass sich die beiden Griffteile 3a, 3b beim Zusammendrücken der Pinzettenschenkel der Pinzette 1 aneinander festsaugen oder miteinander verkleben können. Dem gleichen Zwecke dient der Anschlag 2 (Figur 2), der in einer entsprechenden Oeffnung am anderen Schenkel geführt sein kann. Somit kann das Instrument nicht von selbst geschlossen bleiben, wenn es sich aufgrund seiner Rückstellkräfte von selbst öffnen müsste und der Operateur, insbesondere bei einer Mikrooperation, eine solche Spreizung des Instrumentes wünscht.

Bei diesem Ausführungsbeispiel ist vorgesehen, dass der Handgriff 3 an dem Griffbereich 4 verrastbar ist. An der Pinzette können neben Anschlag 5 und am Handgriff 3 neben der entsprechenden Ausnehmung 6 zur weiteren Verrastung am Griffbereich 4 dieses Instrumentes Einbuchtungen 14 oder Vorsprünge und am Handgriff 3 entsprechende Gegenverformungen 15 vorgesehen sein. Dadurch lässt sich der jeweilige Metallkörper relativ einfach herstellen, da an ihm keine vorstehenden Rastvorsprünge benötigt werden, die schwieriger als Einsenkungen zu fertigen wären. Aufgrund der Elastizität und Weichheit des Werkstoffes des Handgriffes 3 können dessen Verformungen notfalls nachgeben und an die für sie vorbestimmten Stellen gleiten und dort einrasten. Als Werkstoff kommt dabei beispielsweise Silikonkautschuk in Frage, da er sich gut heiss sterilisieren lässt und in der gewünschten Weichheit von unter 80 Shore A z. B. 25 bis 50 Shore A herstellen lässt.

Der Handgriff 3 kann, was Figur 4 verdeutlicht, auch in Drehrichtung formschlüssig, also drehfest mit dem Instrument 1 verbunden werden, so dass ein Benutzer auch Drehkräfte mit grosser Sicherheit auf das Instrument übertragen kann.

In den Figuren 2 und 4 erkennt man noch, dass die Aussenseite des weichen Handgriffes 3 profiliert, insbesondere mit Umfangsrillen 11, gegebenenfalls auch mit Längsrillen oder Mulden versehen sein kann. Dadurch kann die durch die Weichheit des Handgriffes 3 schon beachtliche Griffigkeit weiter verbessert werden.

Der Querschnitt des Handgriffes 3 für das Heftinstrument 2 kann im Querschnitt kreis- oder sternförmig oder vieleckig sein. Dies kommt einer guten Griffigkeit und auch einer guten Drehbarkeit eines solchen Instrumentes entgegen. Für die Verbindung mit dem Metallkörper des Instrumentes ist es vorteilhaft, wenn diese Einsenkungen und der Griffteil in seiner Stecköffnung Vorsprünge hat, die in die Einsenkungen passen.

In überraschender Weise wird also von den bisher relativ harten aufgerauhten Handgriffen abgegangen und die gute Kraftübertragung eines weichen und etwas nachgiebigen Werkstoffes dadurch genutzt, dass der Handgriff aus einem solchen weichen Werkstoff gefertigt ist. Dadurch können nun beliebige, auch relativ grosse Kräfte über den Handgriff auf das Instrument übertragen werden, ohne dass die Gefahr einer Ablösung besteht oder ein Abrutschen zu befürchten ist. Versuche haben gezeigt, dass selbst mit feuchten Operationshandschuhen eine sichere Handhabung des derartig gestalteten Instrumentes möglich ist.

Da aus den vollen Metallteilen vorstehende Handgriffe vermieden werden, wird die Herstellung der Metallteile der jeweiligen Instrumente einfacher, die Instrumente erhalten ein geringeres Gewicht und gleichzeitig werden durch die weichen Handgriffe 3 die Griffigkeit und die Uebertragbarkeit von Operationskräften verbessert. Dabei ist eine sehr einfache Verbindung zwischen Handgriff und Instrument geschaffen, bei der ein separates Vernieten oder Verbinden von Griffteilen und ein Verpressen von Griffteilen aus einem Werkstoff mit Instrumententeilen aus einem anderen Werkstoff vermieden wird.

Vor allem bei Kombination einzelner oder mehrerer der vorgeschriebenen Merkmale und Massnahmen ergibt sich ein chirurgisches Instrument mit einem sehr gut zu handhabenden Handgriff, auf den der Operateur bei seinen verschiedenen Arbeiten und Arbeitsweisen praktisch keine Rücksicht nehmen muss, da sich dieser Handgriff selbst an die ihn bewegende und haltende Hand anpasst und dabei gleichzeitig eine gute Kraftübertragung in beliebigen Richtungen erlaubt. Dennoch ist die Herstellung eines solchen Instrumentes mit einem derartigen Handgriff preiswert und gleichzeitig ergibt sich als zusätzlicher Vorteil, dass ein solches Instrument gegenüber einer Ganzmetallausführung ein geringeres Gewicht hat, wodurch eine wesentlich geringere Ermüdung, insbesondere bei langen Operationen, eintritt.

## Patentansprüche

1. Zweischenkliges chirurgisches Instrument mit beweglichem Arbeitsende, insbesondere Pinzette, mit einem Handgriff (3) aus zwei Hälften (3a, 3b) aus Kunststoff, die die Längsseiten der Instrumentenschenkel (4) einschliessen, und Mitteln (5, 6) zum Ineinander-Verrasten des Handgriffes und des Instrumentes, dadurch gekennzeichnet, dass die Handgriff-Hälften (3a, 3b) aus weichem, heiss sterilisierbaren Kunststoff, insbesondere Silikonkautschuk, bestehen und den Griffbereich (4) der Instrumentenschenkel ganz umschliessen, wobei die Durchstecköffnung (8) der Handgriff-Hälfte (3a, 3b) nahe ihrer innenseitigen Oberfläche (9) verläuft, und die Mittel zum Ineinander-Verrasten des Instrumentes und der Handgriff-Hälften ein Anschlag (5) im Griffbereich (4) des Instrumentes und eine diesem Anschlag entsprechende Ausnehmung (6) in der Einstecköffnung (8) der Handgriff-Hälfte sind.

2. Instrument nach Anspruch 1, dadurch gekennzeichnet, dass der Querschnitt der Handgriff-Hälften (3a, 3b) halbkreisförmig oder oval, sternförmig oder vieleckig ist, und die Handgriff-Hälften formschlüssig mit dem Instrument verbunden sind.

3. Instrument nach Anspruch 1 oder 2, dadurch gekennzeichnet, dass der weiche Handgriff (3) vorgefertigt und auf das Instrument und den Griffbereich (4) aufsteckbar und lösbar mit ihm verbindbar ist.

4. Instrument nach Anspruch 1, dadurch gekennzeichnet, dass der weiche Handgriff (3) am Griffbereich (4) angegossen oder anvulkanisiert ist.

5. Instrument nach einem der Ansprüche 1 bis 3, dadurch gekennzeichnet, dass am Griffbereich (4) Einbuchtungen (14) und/oder Vorsprünge und im Inneren des Handgriffes (3) entsprechende Gegenverformungen (15) vorgesehen sind, die aufgrund der Elastizität und Weichheit des Werkstoffes des Handgriffes (3) beim Aufstecken nachgeben.

6. Instrument nach einem der Ansprüche 1 bis 5, dadurch gekennzeichnet, dass die Härte des Handgriffes (3) unter 80 Shore A, vorzugsweise zwischen 25 bis 50 Shore A liegt.

7. Instrument nach einem der Ansprüche 1 bis 3 oder 5 bis 7, dadurch gekennzeichnet, dass an einer oder beiden der einander zugewandten Innenflächen (9) der Handgriffhälften (3a, 3b) Vorsprünge, Noppen (10) oder dgl. Abstandhalter (2) vorgesehen sind.

8. Instrument nach einem der Ansprüche 1 bis 7, dadurch gekennzeichnet, dass die Aussenseite (7) des weichen Handgriffes (3) profiliert, insbesondere mit Umfangsrillen (11) und/oder Längsrillen oder Mulden versehen ist.

9. Instrument nach einem der Ansprüche 1 bis 8, dadurch gekennzeichnet, dass der jeweilige Schenkel seine Breite und/oder Dicke ändert und die Innenöffnung (8) der Handgriffhälften (3a, 3b) eine entsprechende Form aufweist.

## Claims

1. A surgery instrument comprising two branches with mobile working end, more particularly pincers, with a handle (3) in two halves (3a, 3b) of synthetic material which incorporate the longitudinal sides of the branches (4) of the instrument, and means (5, 6) for fitting together the handle and the instrument, characterized in that the halves (3a, 3b) of the handle are of soft synthetic material, more particularly silicone rubber, capable to be hot sterilized and that they surround entirely the region of the handle (4) of the branches of the instrument, the interlocking opening (8) of the halves (3a, 3b) of the handle being located near their internal surface (9) and the means for fitting together the instrument and the halves of the handle are a stop (5) at the region of the handle (4) of the instrument and a recess (6) corresponding to said stop in the interlocking opening (8) of the halves of the handle.

2. An instrument according to claim 1, characterized in that the section of the halves (3a, 3b) of the handle is semi-circular, oval, star-shaped or polygonal and that the halves of the handle are form fitting connected to the instrument.

3. An instrument according to claim 1 or 2, characterized in that the soft handle (3) is prefabricated and capable to be attachably and removably connected to the instrument and to the region of the handle (4).

4. An instrument according to claim 1, characterized in that the soft handle (3) is casted on or vulcanized at the region of the handle (4).

5. An instrument according to one of the claims 1 to 3, characterized in that indentations (14) and/or projections and complementary formations thereof inside of the handle (3) are provided at the region of the handle (4) which give way whilst attaching because of the elasticity and the softness of the handle.

6. An instrument according to one of the claims 1 to 5, characterized in that the hardness of the handle (3) is lower than 80 Shore A, preferably between 25 and 50 Shore A.

7. An instrument according to one of the claims 1 to 3, 5 or 6, characterized in that projections, nops (10) or similar distance pieces (2) are provided at one or both opposite internal faces (9) of the halves (3a, 3b) of the handle.

8. An instrument according to one of the claims 1 to 7, characterized in that the external side (7) of the soft handle (3) is profiled, more particularly with peripheral grooves (11) and/or longitudinal grooves or hutches.

9. An instrument according to one of the claims 1 to 8, characterized in that the respective branch changes its width and/or thickness and that the internal opening (8) of the halves (3a, 3b) of the handle has a corresponding form.

## Revendications

1. Instrument chirurgical à deux branches avec extrémité de travail mobile, plus particulièrement pincette, avec une poignée (3) en deux moitiés (3a, 3b) en matériau synthétique qui incorporent les côtés longitudinaux des branches (4) de l'instrument, et des moyens (5, 6) pour engager l'un dans l'autre la poignée et l'instrument, caractérisé en ce que les moitiés de poignée (3a, 3b) sont en matériau synthétique tendre, stérilisable à chaud, en particulier en caoutchouc au silicone, et qu'elles entourent entièrement l'endroit de poignée (4) des branches de l'instrument, l'ouverture d'emboîtement (8) des moitiés de poignée (3a, 3b) se situant près de leur surface interne (9) et les moyens pour engager l'un dans l'autre l'instrument et les moitiés de poignée sont une butée (5) à l'endroit de poignée (4) de l'instrument et un dégagement (6) correspondant à cette butée dans l'ouverture d'emboîtement (8) des moitiés de poignée.

2. Instrument selon la revendication 1, caractérisé en ce que la section des moitiés de poignée (3a, 3b) est en demi-cercle ou ovale, en forme d'étoile ou polygonale et que les moitiés de poignée sont connectées à l'instrument d'une manière ajustée à sa forme.

3. Instrument selon la revendication 1 ou 2, caractérisé en ce que la poignée tendre (3) est préfabriquée et susceptible d'être connectée à l'instrument et à l'endroit de poignée (4) de manière emboîtable et détachable.

4. Instrument selon la revendication 1, caractérisé en ce que la poignée tendre (3) est apportée par moulage ou par vulcanisation à l'endroit de poignée (4).

5. Instrument selon l'une des revendications 1 à 3, caractérisé en ce qu'à l'endroit de poignée (4), des indentations (14) et/ou saillies, et à l'intérieur de la poignée (3) des formations complémentaires (15) sont prévues qui, à cause de l'élasticité et de la molesse du matériau de la poignée (3), fléchissent lors de l'emboîtement.

6. Instrument selon l'une des revendications 1 à 5, caractérisé en ce que la dureté de la poignée (3) est inférieure à 80 Shore A, de préférence entre 25 et 50 Shore A.

7. Instrument selon l'une des revendications 1 à 3, 5 ou 6, caractérisé en ce que des saillies, boutons (10) ou des pièces de distance (2) similaires sont prévues à l'une ou aux deux faces intérieures (9) opposées des moitiés de poignée (3a, 3b).

8. Instrument selon l'une des revendications 1 à 7, caractérisé en ce que le côté extérieur (7) de la poignée tendre (3) est profilé, plus particulièrement avec des rainures périphériques (11) et/ou des rainures longitudinales ou des creux.

9. Instruments selon l'une des revendications 1 à 8, caractérisé en ce que la branche respective modifie sa largeur et/ou son épaisseur et que l'ouverture intérieure (8) des moitiés de poignée (3a, 3b) a une forme correspondante.

FIG.1

FIG.2

FIG.3

FIG.4